# EUROPEAN PATENT SPECIFICATION

(11) **EP 3 395 756 B1**
(45) Date of publication and mention of the grant of the patent: **11.12.2019**
(21) Application number: 17168770.0
(22) Date of filing: 28.04.2017
(51) Int. Cl.: B67D 7/32, B67D 7/56, B67D 7/04, B67D 7/08

(54) **A FUEL PUMP UNIT FOR A FUEL DISPENSING UNIT, A FUEL DISPENSING UNIT FOR REFUELLING A VEHICLE, AND A METHOD FOR HANDLING A FUEL PUMP UNIT OF A FUEL DISPENSING UNIT**
KRAFTSTOFFPUMPENEINHEIT FÜR EINE KRAFTSTOFFAUSGABEEINHEIT, KRAFTSTOFFAUSGABEEINHEIT ZUR BETANKUNG EINES FAHRZEUGS UND VERFAHREN ZUR HANDHABUNG EINER KRAFTSTOFFPUMPENEINHEIT EINER KRAFTSTOFFAUSGABEEINHEIT
UNITÉ DE POMPE À CARBURANT POUR UNE UNITÉ DE DISTRIBUTION DE CARBURANT, UNITÉ DE DISTRIBUTION DE CARBURANT POUR RAVITAILLER UN VÉHICULE ET PROCÉDÉ DE GESTION D'UNE UNITÉ DE POMPE À CARBURANT D'UNE UNITÉ DE DISTRIBUTION DE CARBURANT

(43) Date of publication of application: 31.10.2018
(73) Proprietor: Wayne Fueling Systems Sweden AB, 202 15 Malmö (SE)
(72) Inventor: Mårtensson, Mattias, 244 33 Kävlinge (SE)
(74) Representative: AWA Sweden AB

(56) References cited:
- EP-A1- 2 241 533
- WO-A1-02/098787
- WO-A2-94/04286
- JP-A- H09 188 400
- JP-A- 2006 240 727
- JP-A- 2014 025 821
- KR-B1- 101 608 819

## Description

### Technical field

The invention relates to a fuel pump unit for a fuel dispensing unit, a fuel dispensing unit for refuelling a vehicle and a method for handling a fuel pump unit of a fuel dispensing unit.

### Background art

A typical fuel dispensing environment, such as the forecourt of a retail fuel dispensing station, comprises a large number of components both for fuel handling and for conducting fuel dispensing transactions. Examples of such components include fuel dispensers, fuel piping, underground storage tanks, submersible turbine and self-contained pumps, motors, and dispensing nozzles. Further, fuel dispensers themselves typically contain flow meters, pulsers, control electronics, valves, card readers, manifolds, and internal fuel and vapour recovery piping, among others. Many of these components are subject to regulatory requirements to maintain a high degree of accuracy and safety and to guard against environmental impact.

As is well known, for a variety of reasons, these components require periodic maintenance or replacement. Some of these components tend to wear over time, which may cause loss of accuracy or efficiency in a fuelling transaction or other operational issues. Component wear can be caused by manufacturing defects, poor fuel quality, or excessive use, among other causes. Eventually, the components may fail (e.g., failure of a pump motor or a leak in the fuel piping) leading to downtime while the components are replaced. Further, some of the components may fail to operate properly, leading to customer frustration or the inability to complete a fuelling transaction.

KR 101 608 819 B1 discloses an oil leakage detection device for a gas station, and an oil leakage detection system for a gas station. The oil leakage detection device is an oil transfer pipe transferring oil of an oil storage tank to a lubricator. The oil transfer pipe comprises a first pipe fastened to the oil storage tank and a second pipe fastened to one end of the first pipe; a suction pump connected between one end of the second pipe and the lubricator to suck the oil from the oil storage tank; an ultrasonic sensor attached to an outer wall of the second pipe by a non-destructive method to generate an ultrasonic wave inside the second pipe, and receiving the ultrasonic wave via the inside of the second pipe; and an oil leakage determination unit analyzing a signal of the ultrasonic sensor to detect whether or not there is an oil leakage inside the second pipe.

A common failure of high flow fuel systems is cavitation. The combination of too much heat or too much inlet restriction can create this operating condition, in which the liquid fuel literally vaporizes (boils) inside the pump assembly. Symptoms of this operating condition may include one or more of the following; dramatic loss of flow rate, gauge bouncing, ratchet or grinding sounds from pump, inconsistent or loss of fuel pressure and temperatures above 50°C.

Loss of fuel delivery pressure will also result in a lean-out condition, and may also result in engine damage. Typically, even with very short time periods of exposure to cavitation (depending on severity) may cause damage to the fuel pump. Such damage results in a direct loss of capacity and efficiency. Eventually, the fuel system will not be able to build or maintain pressure.

### Summary of the invention

It is an objective of the present invention to provide an improvement of the above technique and prior art. More particularly, it is an objective of this invention to provide an improved fuel pump unit with a decreased energy consumption.

According to a first aspect, these and other objects, and/or advantages that will be apparent from the following description of embodiments, are achieved, in full or at least in part, by a fuel pump unit for a fuel dispensing unit according to the subject-matter of independent claim 1.

This is advantageous in that a potential problem relating to a cavitation issued or the presence of air bubbles in fuel flow can be detected in a simple and reliable way. The solution is also cost efficient compared to the solutions available on the market today.

The at least one ultrasonic transmitter and the at least one ultrasonic receiver may be arranged on one side of the fuel flow path.

The at least one ultrasonic transmitter and the at least one ultrasonic receiver may be constituted by one single element. Here, the single element may comprise a piezo element.

The at least one ultrasonic transmitter and the at least one ultrasonic receiver may be comprised in one single unit.

The at least one ultrasonic transmitter and the at least one ultrasonic receiver may be arranged on opposite sides of the fuel flow path.

The fuel pump unit may comprise a first ultrasonic transmitter and a first ultrasonic receiver arranged on one side of the fuel flow path, and a second ultrasonic transmitter and a second ultrasonic receiver arranged on the other side of the fuel flow path. Further, the first ultrasonic transmitter and the first ultrasonic receiver may be constituted by one single element, and the second ultrasonic transmitter and the second ultrasonic receiver may be constituted by one single element. Here, the single element may comprise a piezo element.

The at least one parameter may be constituted by a time period, a frequency of the ultrasonic signal and/or an amplitude of the ultrasonic signal.

The control unit may be adapted to report that problem has been identified if the difference in the at least one parameter is above a threshold value.

The fuel pump unit may further comprise a bypass channel arranged between the pressure side and the suction side of the pump, wherein the bypass channel is provided with a control valve.

The at least one ultrasonic transmitter and the at least one ultrasonic receiver may be arranged upstream of the bypass channel at the suction side of the pump.

According to a second aspect, these and other objects are achieved, in full or at least in part, by a fuel dispensing unit for refuelling a vehicle, which comprises a fuel pump unit according to the features described above.

According to a third aspect, these and other objects are achieved, in full or at least in part, by a method for handling a fuel pump unit for a fuel dispensing unit according to the subject-matter of independent claim 15.

The specific action may for example be constituted by indicating that a problem with the pump has been identified, stopping the pump or evaluating if the difference may depend on cavitation of the fuel at the suction side of the pump or a leak in the fuel flow path.

Effects and features of the second and third aspect of the present invention are largely analogous to those described above in connection with the first aspect of the inventive concept. Embodiments mentioned in relation to the first aspect of the present invention are largely compatible with the further aspects of the invention.

Other objectives, features and advantages of the present invention will appear from the following detailed disclosure, from the attached claims, as well as from the drawings.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of said element, device, component, means, step, etc., unless explicitly stated otherwise.

As used herein, the term "comprising" and variations of that term are not intended to exclude other additives, components, integers or steps.

### Brief description of the drawings

The above, as well as additional objects, features and advantages of the present invention, will be better understood through the following illustrative and non-limiting detailed description of embodiments of the present invention, with reference to the appended drawings, where the same reference numerals may be used for similar elements, and wherein:
Fig. 1 is a schematic view of an exemplary embodiment of a fuel dispensing unit according to a second aspect of the invention.
Fig. 2 is a cross sectional view of an exemplary embodiment of a fuel pump unit according to a first aspect of the invention for the fuel dispensing unit in Fig. 1.
Fig. 3 is a cross sectional view of another exemplary embodiment of a fuel pump unit according to the first aspect of the invention for the fuel dispensing unit in Fig. 1.
Fig. 4 is a cross sectional view of yet another exemplary embodiment of a fuel pump unit according to the first aspect of the invention for the fuel dispensing unit in Fig. 1.
Fig. 5 is a cross sectional view of yet another exemplary embodiment of a fuel pump unit according to the first aspect of the invention for the fuel dispensing unit in Fig. 1.

### Detailed description of preferred embodiments of the invention

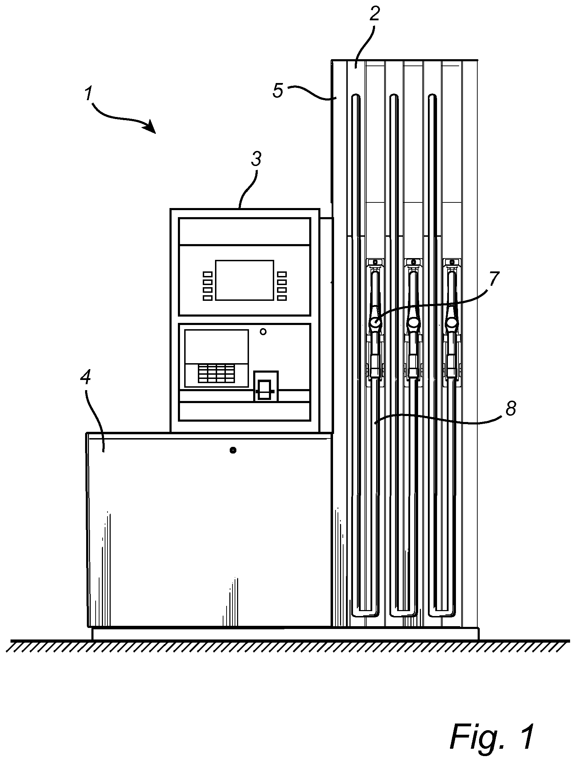

Fig. 1 illustrates an exemplary fuel dispensing unit 1, having hose storage spaces 2 on each opposing side of the fuel dispensing unit 1, an electrical cabinet 3 containing all the electronics for the fuel dispensing unit 1, a hydraulic cabinet 4 containing fuel dispensing means (not shown), e.g. fuel metering means, valves, vapour recovery system etc., and a column 5 extending vertically between and separating the electrical cabinet 3 and the hydraulic cabinet 4 from the hose storage spaces 2.

The fuel dispensing unit 1 is connected to an underground reservoir (not shown) containing fuel. When filling up the tank of a motor vehicle, the fuel is pumped from the underground reservoir by means of a pump 6 which is located in the hydraulic cabinet 4, and from there to the column 5 and out to a nozzle 7 via a hose 8.

A main fuel supply pipe 9 extends between the underground reservoir and the suction side S of the pump 6, while the hose 8 extends between the pressure side P of the pump 6 and the nozzle 7. The suction side S provides an underpressure to the main fuel supply pipe 9 and the pressure side S provides an overpressure to the hose 8.

The fuel dispensing unit 1 has a nozzle boot 13 in which the nozzle 7 is arranged when not in use. The nozzle boot 13 preferably comprises a sensor (not shown) for detecting if the nozzle 7 is present in the nozzle boot 13. Further, the nozzle 7 is normally equipped with a flow meter (not shown) for measuring the fuel flow rate from the nozzle 7 upon refuelling.

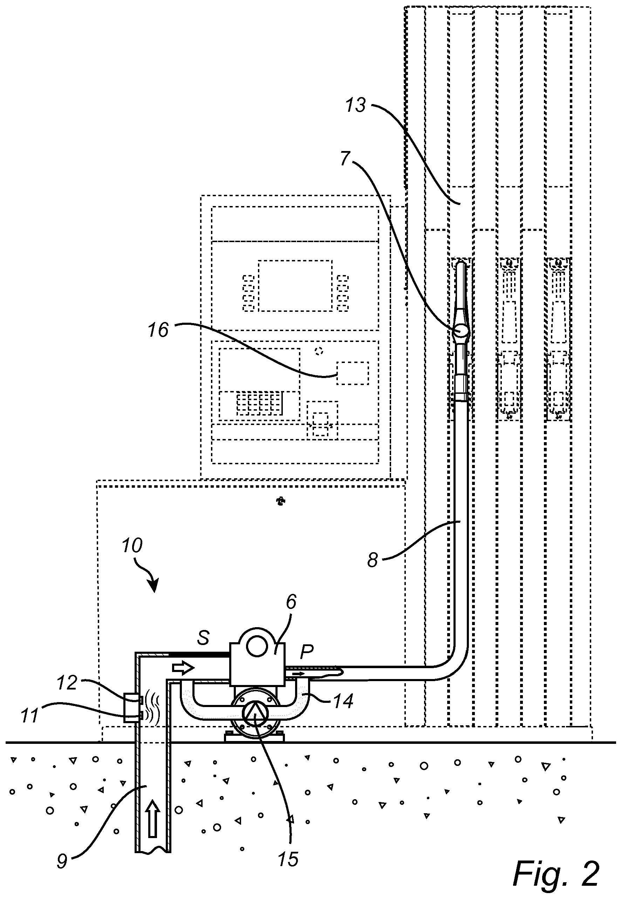

In Fig. 2, an exemplary embodiment of a fuel pump unit 10 for the fuel dispensing unit 1 is illustrated. The fuel pump unit 10 comprises the pump 6 with the suction side S and the pressure side P for pumping fuel along a fuel flow path established between the underground reservoir and the nozzle 7, an ultrasonic transmitter 11 adapted to send out an ultrasonic signal in the fuel flow path, and an ultrasonic receiver 12 adapted to receive the ultrasonic signal. The ultrasonic transmitter 11 and the ultrasonic receiver 12 are comprised in one single unit, which is arranged at the suction side S of the pump 6.

In this embodiment, the pump 6 comprises a bypass channel 14 arranged between the pressure side P and the suction side S of the pump 6. The bypass channel 14 has a control valve 15 which is constituted by a spring valve. When the pump 6 is equipped with such a bypass channel 14, the ultrasonic transmitter 11 and the ultrasonic receiver 12 are arranged upstream of the bypass channel 14 at the suction side S of the pump 6.

The fuel pump unit 10 further comprises a control unit 16 connected to the ultrasonic transmitter 11 and to the ultrasonic receiver 12. The control unit 16 is adapted to detect a difference in at least one parameter based on a comparison between the ultrasonic signal and a reference signal.

The parameter to be analysed by the control unit 16 can be the time period it takes for the ultrasonic signal to travel from the ultrasonic transmitter 11 to the ultrasonic receiver 12 in the fuel flow path. It can also be the actual frequency or the amplitude of the ultrasonic signal as well as a combination of all of the above.

The reference signal can be constituted by a predetermined signal which for example is read from a table based on the media present in the fuel flow path when the ultrasonic signal is sent. It can also be constituted by an actual measurement conducted in the same manner as described above but at an earlier or later stage, for example upon calibration of the fuel pump unit 10.

Upon detection of a problem with the fuel pump unit 10, the control unit 16 can be programmed to conduct a specific action. The specific action may for example be constituted by indicating that a problem with the pump 6 has been identified, stopping the pump 6 or evaluating if the difference depends on cavitation of the fuel at the suction side S of the pump 6.

There are numerous ways of using the information retrieved from the ultrasonic signal in order to detect potential problems in the fuel pump unit 10, and naturally, other values than time, frequency and amplitude can be used as a parameter for drawing conclusions relating to the condition of the fuel pump unit 10 based on information retrieved from the ultrasonic transmitter 11 and the ultrasonic receiver 12.

In one example not claimed, the ultrasonic transmitter 11 and the ultrasonic receiver 12 are used to detect the volume of the fuel present in the main fuel supply pipe 9 when the fuel dispensing unit 1 not is in operation. Based on the volume of the fuel detected by the ultrasonic transmitter 11 and the ultrasonic receiver 12, the control unit 16 is able to calculate the density of the fuel. The corresponding calculations is later on conducted when the fuel dispensing unit 1 is in operation. Thereafter, the calculated fuel density values can be compared with each other to determine any potential deviations.

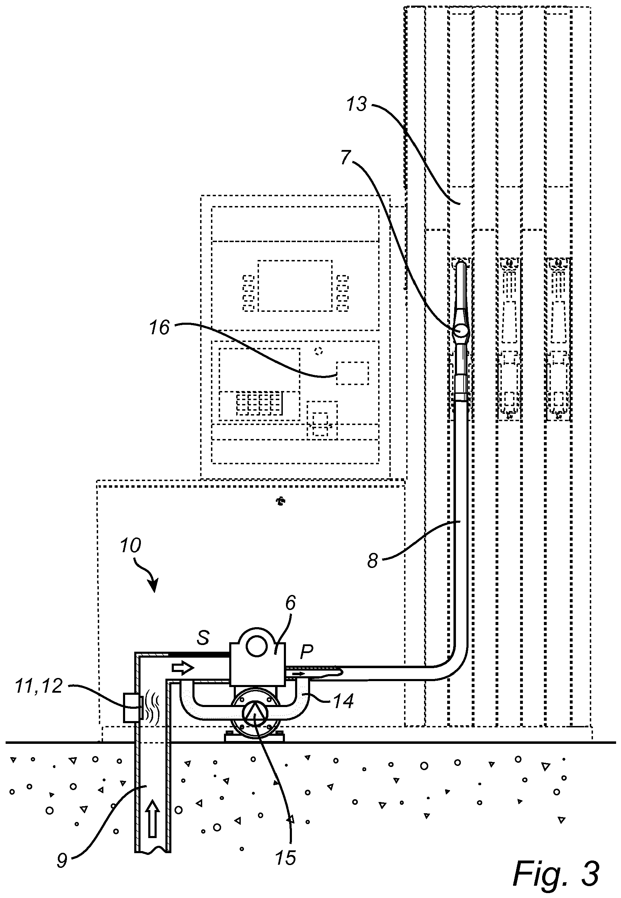

In Fig. 3, another exemplary embodiment of the fuel pump unit 10 for the fuel dispensing unit 1 is illustrated. In this embodiment, ultrasonic transmitter 11 and the ultrasonic receiver 12 are constituted by one single element which comprises a piezo element.

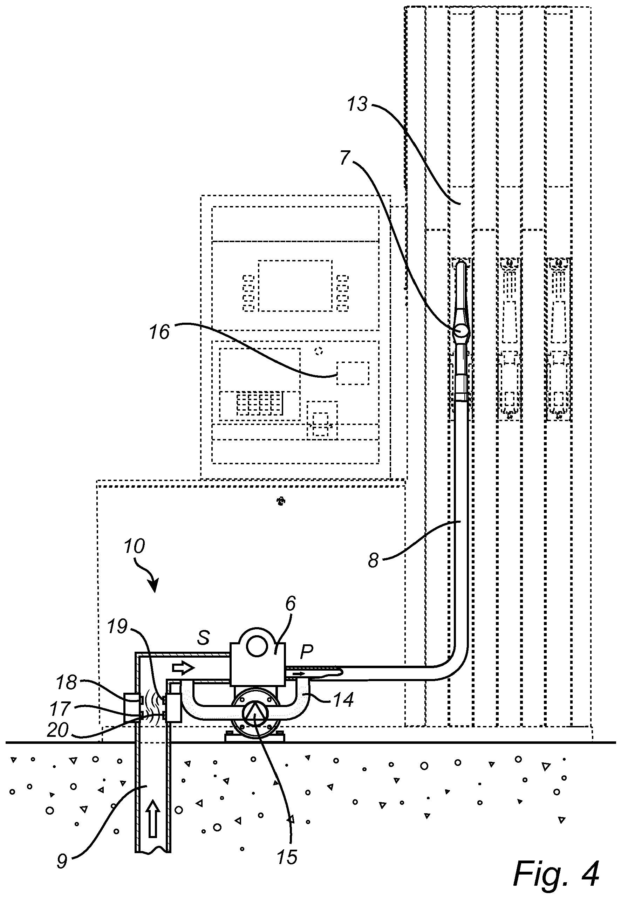

Fig. 4 illustrates another exemplary embodiment of the fuel pump unit 10 for the fuel dispensing unit 1. In this embodiment, the fuel pump unit 10 comprises a first ultrasonic transmitter 17 and a first ultrasonic receiver 18 arranged on one side of the fuel flow path, and a second ultrasonic transmitter 19 and a second ultrasonic receiver 20 arranged on the other side of the fuel flow path. The first ultrasonic transmitter 17 and the first ultrasonic receiver 18 are comprised in one single unit, and the second ultrasonic transmitter 19 and the second ultrasonic receiver 20 are comprised in one single unit.

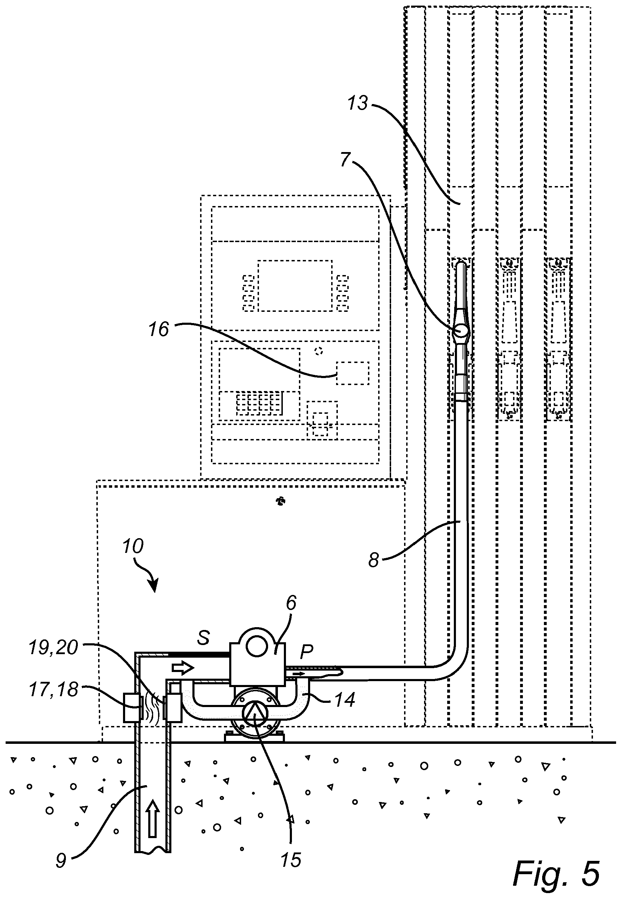

Fig. 5 illustrates another exemplary embodiment of the fuel pump unit 10 for the fuel dispensing unit 1. In this embodiment, the fuel pump unit 10 comprises the first ultrasonic transmitter 17 and the first ultrasonic receiver 18 which are arranged on one side of the fuel flow path and constituted by one single piezo element, and the second ultrasonic transmitter 19 and the second ultrasonic receiver 20 which are arranged on the other side of the fuel flow path constituted by one single piezo element.

One exemplary method of handling the fuel pump unit 10 in the fuel dispensing unit 1 in order to detect any potential problems relating to a cavitation issued or the presence of air bubbles in fuel flow will be described in detail below with reference to the embodiment illustrated in Fig. 2.

When the nozzle 7 is removed from the nozzle boot 13, the control unit 16 is alerted and a pump motor (not shown) driving the pump 6 is started. The pump motor is operated at a fixed speed, and therefore, the fuel is circulated from the pressure side P to the suction side S via the bypass channel 14 at full capacity (for example at a rate of 80 litres/minute) until the actual refuelling of a vehicle has started. When the nozzle 7 is opened and the fuel starts flowing therefrom, a pressure drop will occur and the pressure from the fuel applied on the spring valve 15 in the bypass channel 14 will decrease. In other words, if the nozzle 7 starts to deliver fuel to a vehicle at a flow rate of 40 litres/minute, the fuel flow rate through the bypass channel 14 will decrease to 40 litres/minute.

In the embodiment illustrated in Fig. 2, the ultrasonic transmitter 11 and the ultrasonic receiver 12 are comprised in one single unit, which is arranged at the suction side S of the pump 6. When a time period is used as the comparison parameter, the control unit 16 will detect the time period it takes for the ultrasonic signal to travel through the media in the fuel flow path from the ultrasonic transmitter 11, via the inner wall of the main supply pipe 9, and back to the ultrasonic receiver 12. That time period is thereafter compared with the corresponding time period of a reference signal.

Specifically, the detected time period can for example be compared with a targeted time period stored in the control unit 16 or read from a table. It can also be compared with a detected time period of a reference signal which is conducted in the same manner as described above upon calibration of the fuel pump unit 10. Should the difference between the time period of the ultrasonic signal and the reference signal deviate more than a predetermined error margin it can be concluded that there is a problem with the fuel pump unit 10.

A problem as referred to above is often related to fuel cavitation or leakage upstream of the ultrasonic transmitter 11 and the ultrasonic receiver 12. Leaks can occur, for example, in and among the components and interconnections which form the fluid paths, e.g., from the undergound reservoir to the nozzle 7. These leaks can allow air to intrude into the fuel from outside the surroundings as the fuel pump unit 10 builds vacuum in the fluid flow path. Moreover, leaks can result in loss of vacuum in the fluid paths when the fuel pump unit 10 is idle (e.g., not active between customers). This loss of vacuum during idle-time can extend the time necessary to dispense fuel, as the fuel pump unit 10 will need to re-build lost pressure.

The skilled person realizes that a number of modifications of the embodiments described herein are possible without departing from the scope of the invention, which is defined in the appended claims.

For instance, other potential parameters than time that could be used are the frequency of the ultrasonic signal (Doppler effect) and the amplitude of the response signal of the transmitted ultrasonic signal.

Further, the ultrasonic signal can be transmitted from the ultrasonic transmitter 11, through the media in the fuel flow path, and directly to the ultrasonic receiver 12. In other embodiments, the ultrasonic signal will bounce of a wall or the like before reaching the ultrasonic receiver 12.

## Claims

1. A fuel pump unit (10) for a fuel dispensing unit (1), comprising
a pump (6) with a suction side (S) and a pressure side (P) for pumping fuel along a fuel flow path between an underground reservoir and a nozzle (7) of the fuel dispensing unit (1),
at least one ultrasonic transmitter (11) arranged at the suction side (S) of the pump (6) and adapted to send out an ultrasonic signal in the fuel flow path, and
at least one ultrasonic receiver (12) arranged at the suction side (S) of the pump (6) and adapted to receive the ultrasonic signal,
**characterised in that** the fuel pump unit further comprises
a control unit (16) connected to the at least one ultrasonic transmitter (11) and the at least one ultrasonic receiver (12), and adapted to detect air bubbles in the fuel flow by detecting a difference in at least one parameter based on a comparison between the ultrasonic signal and a reference signal.

2. The fuel pump unit (10) according to claim 1, wherein the at least one ultrasonic transmitter (11) and the at least one ultrasonic receiver (12) are arranged on one side of the fuel flow path.

3. The fuel pump unit (10) according to claim 1 or 2, wherein the at least one ultrasonic transmitter (11) and the at least one ultrasonic receiver (12) are constituted by one single element, the single element comprising a piezo element.

4. The fuel pump unit (10) according to any one of the preceding claims, wherein the at least one ultrasonic transmitter (11) and the at least one ultrasonic receiver (12) are comprised in one single unit.

5. The fuel pump unit (10) according to claim 1, wherein the at least one ultrasonic transmitter (11) and the at least one ultrasonic receiver (12) are arranged on opposite sides of the fuel flow path.

6. The fuel pump unit (10) according to claim 1, wherein the fuel pump unit (10) comprises a first ultrasonic transmitter (17) and a first ultrasonic receiver (18) arranged on one side of the fuel flow path, and a second ultrasonic transmitter (19) and a second ultrasonic receiver (20) arranged on the other side of the fuel flow path.

7. The fuel pump unit (10) according to claim 6, wherein the first ultrasonic transmitter (17) and the first ultrasonic receiver (18) are constituted by one single element, and the second ultrasonic transmitter (19) and the second ultrasonic receiver (20) are constituted by one single element, the single element comprising a piezo element.

8. The fuel pump unit (10) according to any one of the preceding claims, wherein the at least one parameter is constituted by a time period.

9. The fuel pump unit (10) according to any one of the preceding claims, wherein the at least one parameter is constituted by a frequency of the ultrasonic signal.

10. The fuel pump unit (10) according to any one of the preceding claims, wherein the at least one parameter is constituted by an amplitude of the ultrasonic signal.

11. The fuel pump unit (10) according to any one of the preceding claims, wherein the control unit (16) is adapted to report that problem has been identified if the difference in the at least one parameter is above a threshold value.

12. The fuel pump unit (10) according to any one of the preceding claims, further comprising a bypass channel (14) arranged between the pressure side (P) and the suction side (S) of the pump, the bypass channel (14) being provided with a control valve (15).

13. The fuel pump unit (10) according to claim 12, wherein the at least one ultrasonic transmitter (11) and the at least one ultrasonic receiver (12) are arranged upstream of the bypass channel at the suction side (S) of the pump (6).

14. A fuel dispensing unit (1) for refuelling a vehicle, comprising a fuel pump unit (10) according to any one of the claims 1-13.

15. A method for handling a fuel pump unit (10) for a fuel dispensing unit (1), the fuel pump unit (10) comprising
a pump (6) with a suction side (S) and a pressure side (P) for pumping fuel along a fuel flow path between an underground reservoir and a nozzle (7) of the fuel dispensing unit (1),
at least one ultrasonic transmitter (11) arranged at the suction side (S) of the pump (6) and adapted to send out an ultrasonic signal in the fuel flow path,
at least one ultrasonic receiver (12) arranged at the suction side (S) of the pump (6) and adapted to receive the ultrasonic signal, and
a control unit (16) connected to the at least one ultrasonic transmitter (11) and the at least one ultrasonic receiver (12),
**characterised in that** the method comprises
detecting air bubbles in the fuel flow by detecting a difference in at least one parameter based on a comparison between the ultrasonic signal and a reference signal, and conducting a specific action if the difference in the at least one parameter is above a threshold value.

## Patentansprüche

1. Kraftstoffpumpeneinheit (10) für eine Kraftstoffausgabeeinheit (1), umfassend
eine Pumpe (6) mit einer Saugseite (S) und einer Druckseite (P) zum Pumpen von Kraftstoff entlang eines Kraftstoffströmungswegs zwischen einem unterirdischen Speicher und einer Düse (7) der Kraftstoffausgabeeinheit (1),
mindestens einen Ultraschallsender (11), der an der Saugseite (S) der Pumpe (6) angeordnet ist und angepasst ist, um ein Ultraschallsignal in dem Kraftstoffströmungsweg auszusenden, und
mindestens einen Ultraschallempfänger (12), der an der Saugseite (S) der Pumpe (6) angeordnet ist und angepasst ist, um das Ultraschallsignal zu empfangen,
**dadurch gekennzeichnet, dass** die Kraftstoffpumpeneinheit ferner
eine Steuereinheit (16) umfasst, die mit dem mindestens einen Ultraschallsender (11) und dem mindesten einen Ultraschallempfänger (12) verbunden ist, und dazu angepasst ist, um Luftblasen im Kraftstoffstrom durch Erfassen einer Differenz in mindestens einem Parameter basierend auf einem Vergleich zwischen dem Ultraschallsignal und einem Referenzsignal zu erkennen.

2. Kraftstoffpumpeneinheit (10) nach Anspruch 1, wobei der mindestens
eine Ultraschallsender (11) und der mindestens eine Ultraschallempfänger (12) auf einer Seite des Kraftstoffströmungswegs angeordnet sind.

3. Kraftstoffpumpeneinheit (10) nach Anspruch 1 oder 2, wobei der mindestens ein Ultraschallsender (11) und der mindestens eine Ultraschallempfänger (12) durch ein einziges Element gebildet sind, wobei das einzige Element ein Piezoelement umfasst.

4. Kraftstoffpumpeneinheit (10) nach einem der vorhergehenden Ansprüche, wobei der mindestens eine Ultraschallsender (11) und der mindestens eine Ultraschallempfänger (12) in einer einzigen Einheit enthalten sind.

5. Kraftstoffpumpeneinheit (10) nach Anspruch 1, wobei der mindestens eine Ultraschallsender (11) und der mindestens eine Ultraschallempfänger (12) an gegenüberliegenden Seiten des Kraftstoffströmungswegs angeordnet sind.

6. Kraftstoffpumpeneinheit (10) nach Anspruch 1, wobei die Kraftstoffpumpeneinheit (10) auf einer Seite des Kraftstoffströmungswegs einen ersten Ultraschallsender (17) und einen ersten Ultraschallempfänger (18) umfasst, und auf der anderen Seite des Kraftstoffströmungswegs ein zweiter Ultraschallsender (19) und ein zweiter Ultraschallempfänger (20) angeordnet sind.

7. Kraftstoffpumpeneinheit (10) nach Anspruch 6, wobei der erste Ultraschallsender (17) und der erste Ultraschallempfänger (18) durch ein einziges Element gebildet sind, und der zweite Ultraschallsender (19) und der zweite Ultraschallempfänger (20) durch ein einziges Element gebildet sind, wobei das einzelne Element ein Piezoelement umfasst.

8. Kraftstoffpumpeneinheit (10) nach einem der vorhergehenden Ansprüche, wobei der mindestens eine Parameter durch eine Zeitperiode gebildet ist.

9. Kraftstoffpumpeneinheit (10) nach einem der vorhergehenden Ansprüche, wobei der mindestens eine Parameter durch eine Frequenz des Ultraschallsignals gebildet ist.

10. Kraftstoffpumpeneinheit (10) nach einem der vorhergehenden Ansprüche, wobei der mindestens eine Parameter eine Amplitude des Ultraschallsignales ist.

11. Kraftstoffpumpeneinheit (10) nach einem der vorhergehenden Ansprüche, wobei die Steuereinheit (16) angepasst ist, um zu melden, dass ein Problem festgestellt wurde, wenn die Differenz in dem mindestens einen Parameter über einem Schwellenwert liegt.

12. Kraftstoffpumpeneinheit (10) nach einem der vorhergehenden Ansprüche, ferner umfassend einen Bypasskanal (14), der zwischen der Druckseite (P) und der Saugseite (S) der Pumpe angeordnet ist, wobei der Bypasskanal (14) mit einem Steuerventil (15) versehen ist.

13. Kraftstoffpumpeneinheit (10) nach Anspruch 12, wobei der mindestens eine Ultraschallsender (11) und der mindestens eine Ultraschallempfänger (12) vor dem Bypasskanal an der Saugseite (S) der Pumpe (6) angeordnet sind.

14. Kraftstoffausgabeeinheit (1) zur Betankung eines Fahrzeuges, umfassend eine Kraftstoffpumpeneinheit (10) nach einem der Ansprüche 1-13.

15. Verfahren zur Handhabung einer Kraftstoffpumpeneinheit (10) einer Kraftstoffausgabeeinheit (1), wobei die Kraftstoffpumpeneinheit (10) umfasst
eine Pumpe (6) mit einer Saugseite (S) und einer Druckseite (P) zum Pumpen von Kraftstoff entlang eines Kraftstoffströmungswegs zwischen einem unterirdischen Speicher und einer Düse (7) der Kraftstoffausgabeeinheit (1),
mindestens einen Ultraschallsender (11), der an der Saugseite (S) der Pumpe (6) angeordnet ist und angepasst ist, um ein Ultraschallsignal im Kraftstoffströmungsweg auszusenden,
mindestens einen Ultraschallempfänger (12), der an der Saugseite (S) der Pumpe (6) angeordnet ist und angepasst ist, um das Ultraschallsignal zu empfangen, und
eine Steuereinheit (16) die mit dem mindestens einen Ultraschallsender (11) und dem mindestens einen Ultraschallempfänger (12) verbunden ist,
**dadurch gekennzeichnet, dass** das Verfahren umfasst
Erkennen von Luftblasen im Kraftstoffstrom durch Erfassen einer Differenz in mindestens einem Parameter basierend auf einem Vergleich zwischen dem Ultraschallsignal und einem Referenzsignal, und
Durchführen einer spezifischen Aktion, wenn die Differenz des mindestens einen Parameters über einem Schwellenwert liegt.

## Revendications

1. Unité de pompe à carburant (10) pour une unité de distribution de carburant (1), comprenant
une pompe (6) avec un côté aspiration (S) et un côté pression (P) pour pomper du carburant le long d'un trajet d'écoulement de carburant entre un réservoir enterré et un injecteur (7) de l'unité de distribution de carburant (1),
au moins un transmetteur à ultrasons (11) agencé sur le côté aspiration (S) de la pompe (6) et adapté pour envoyer un signal à ultrasons dans le trajet d'écoulement de carburant, et
au moins un récepteur à ultrasons (12) agencé sur le côté aspiration (S) de la pompe (6) et adapté pour recevoir le signal à ultrasons,
**caractérisée en ce que** l'unité de pompe à carburant comprend en outre
une unité de commande (16) raccordée à l'au moins un transmetteur à ultrasons (11) et l'au moins un récepteur à ultrasons (12) et adaptée pour détecter des bulles d'air dans l'écoulement de carburant par la détection d'une différence dans au moins un paramètre en se basant sur une comparaison entre le signal à ultrasons et un signal de référence.

2. Unité de pompe à carburant (10) selon la revendication 1, dans laquelle l'au moins un transmetteur à ultrasons (11) et l'au moins un récepteur à ultrasons (12) sont agencés sur un côté du trajet d'écoulement de carburant.

3. Unité de pompe à carburant (10) selon la revendication 1 ou 2, dans laquelle l'au moins un transmetteur à ultrasons (11) et l'au moins un récepteur à ultrasons (12) sont constitués d'un seul élément, le seul élément comprenant un élément piézo.

4. Unité de pompe à carburant (10) selon l'une quelconque des revendications précédentes, dans laquelle l'au moins un transmetteur à ultrasons (11) et l'au moins un récepteur à ultrasons (12) sont compris dans une seule unité.

5. Unité de pompe à carburant (10) selon la revendication 1, dans laquelle l'au moins un transmetteur à ultrasons (11) et l'au moins un récepteur à ultrasons (12) sont agencés sur des côtés opposés du trajet d'écoulement de carburant.

6. Unité de pompe à carburant (10) selon la revendication 1, dans laquelle l'unité de pompe à carburant (10) comprend un premier transmetteur à ultrasons (17) et un premier récepteur à ultrasons (18) agencés sur un côté du trajet d'écoulement de carburant, et un second transmetteur à ultrasons (19) et un second récepteur à ultrasons (20) agencés sur l'autre côté du trajet d'écoulement de carburant.

7. Unité de pompe à carburant (10) selon la revendication 6, dans laquelle le premier transmetteur à ultrasons (17) et le premier récepteur à ultrasons (18) sont constitués d'un seul élément, et le second transmetteur à ultrasons (19) et le second récepteur à ultrasons (20) sont constitués d'un seul élément, le seul élément comprenant un élément piézo.

8. Unité de pompe à carburant (10) selon l'une quelconque des revendications précédentes, dans laquelle l'au moins un paramètre est constituée d'une durée.

9. Unité de pompe à carburant (10) selon l'une quelconque des revendications précédentes, dans laquelle l'au moins un paramètre est constitué d'une fréquence du signal à ultrasons.

10. Unité de pompe à carburant (10) selon l'une quelconque des revendications précédentes, dans laquelle l'au moins un paramètre est constitué par une amplitude du signal à ultrasons.

11. Unité de pompe à carburant (10) selon l'une quelconque des revendications précédentes, dans laquelle l'unité de commande (16) est adaptée pour rapporter qu'un problème a été identifié si la différence dans l'au moins un paramètre est au-dessus d'une valeur seuil.

12. Unité de pompe à carburant (10) selon l'une quelconque des revendications précédentes, comprenant en outre un canal de dérivation (14) agencé entre le côté pression (P) et le côté aspiration (S) de la pompe, le canal de dérivation (14) étant doté d'un clapet de commande (15).

13. Unité de pompe à carburant (10) selon la revendication 12, dans laquelle l'au moins un transmetteur à ultrasons (11) et l'au moins un récepteur à ultrasons (12) sont agencés en amont du canal de dérivation sur le côté aspiration (S) de la pompe (6).

14. Unité de distribution de carburant (1) pour ravitailler un véhicule comprenant une unité de pompe à carburant (10) selon l'une quelconque des revendications 1 - 13.

15. Procédé de gestion d'une unité de pompe à carburant (10) pour une unité de distribution de carburant (1), l'unité de pompe à carburant (10) comprenant
une pompe (6) avec un côté aspiration (S) et un côté pression (P) pour pomper du carburant le long d'un trajet d'écoulement de carburant entre un réservoir enterré et un injecteur (7) de l'unité de distribution de carburant (1),
au moins un transmetteur à ultrasons (11) agencé sur le côté aspiration (S) de la pompe (6) et adapté pour envoyer un signal à ultrasons dans le trajet d'écoulement de carburant, et
au moins un récepteur à ultrasons (12) agencé sur le côté aspiration (S) de la pompe (6) et adapté pour recevoir le signal à ultrasons,
une unité de commande (16) raccordée à l'au moins un transmetteur à ultrasons (11) et l'au moins un récepteur à ultrasons (12)
**caractérisé en ce que** le procédé comprend de
détecter des bulles d'air dans l'écoulement de carburant par la détection d'une différence dans au moins un paramètre en se basant sur une comparaison entre le signal à ultrasons et un signal de référence, et
mener une action spécifique si la différence dans l'au moins un paramètre est au-dessus d'une valeur seuil.
